# EUROPEAN PATENT SPECIFICATION

(11) **EP 4 226 876 B1**
(45) Date of publication and mention of the grant of the patent: **23.04.2025**
(21) Application number: 22155812.5
(22) Date of filing: 09.02.2022
(51) Int. Cl.: A61B 17/225, A61B 17/22, A61B 6/00, A61B 6/12, A61B 6/04, A61B 17/00, A61B 90/00

(54) **SHOCK WAVE DEVICE HAVING IMPROVED ACOUSTIC COUPLING**
STOSSWELLENVORRICHTUNG MIT VERBESSERTER AKUSTISCHER KOPPLUNG
DISPOSITIF À ONDES DE CHOC AYANT UN COUPLAGE ACOUSTIQUE AMÉLIORÉ

(43) Date of publication of application: 16.08.2023
(73) Proprietor: Storz Medical AG, 8274 Tägerwilen (CH)
(72) Inventor: STORZ, Rafael, 8280 Kreuzlingen (CH); MARLINGHAUS, Ernst, 8598 Bottighofen (CH); GÖRNER, Georg, 8597 Landschlacht (CH)
(74) Representative: Lohr, Jöstingmeier & Partner

(56) References cited:
- EP-A1- 2 628 456
- EP-A1- 3 875 048
- WO-A2-2008/048708
- US-A1- 2010 080 349
- US-A1- 2019 350 685

## Description

### Field of the invention

The invention relates to an extracorporeal shock wave or ultrasound therapy system, e.g., a lithotripsy system or Lithotripter for non-invasive treatment of stones, like e.g., kidney stones, urinary stones, gallstones, or other calculi within a mammal's body using acoustic pulses, or other applications of an extracorporeal shock wave or ultrasound therapy system.

### Description of the related art

Lithotripters which generate high energy pulses to disintegrate concrements in a human body may have shock wave and/or ultrasound sources within a reflector filled with water. A cushion may be provided to enclose the water and to adapt to a separation film, also called patient film or the body of a patient. There may be small air bubbles within a coupling medium, e.g., an ultrasound gel, at an exterior surface of the cushion between the cushion and a patient film or the body of the patient. Such air bubbles may cause reflection and/or refraction of the high energy pulses and therefore may reduce the efficiency of treatment.

DE 10 2005 039 178 A1 discloses a device for detecting such bubbles with a camera system. DE 10 2010 061 852 A1 discloses an illumination device, which detects reflected light at the cushion to indicate air bubbles. US 2010/080349 A1 discloses a breast biopsy device. EP 3 875 048 A1 discloses a shockwave therapy system with 3D control.

### Summary of the invention

The problem to be solved by the invention is to provide an ultrasound and/or shock wave device, which can avoid and/or remove air bubbles at the exterior surface of a cushion of a lithotripsy device covering an ultrasound and/or shock-wave source. A further aspect relates to a method of removing remove air bubbles at the exterior surface of the cushion.

Solutions of the problem are described in the independent claims which define the system and methods according to the invention. The dependent claims relate to further improvements of the invention.

In an embodiment, a shock wave and/or ultrasound device, which may be a lithotripter includes an ultrasound and/or shockwave source within a reflector and covered by a cushion. The interior of the reflector and the cushion may be filled with a liquid, which may be water. The cushion may have an exit section through which ultrasound and/or shockwaves pass to the exterior. This exit section may be directly coupled to a patient or indirectly via a separation film, also called patient film to the patient. The patient film may include a plastic material. There may be a layer of coupling gel, also known as ultrasound gel or acoustic gel at the top of the exit section to allow further coupling of the ultrasound and/or shock-wave energy via the patient film or directly to the patient.

The ultrasound and/or shockwave source is suspended on a hexapod drive, such that it can be displaced and tilted. Displacement may be in at least one and up to three degrees of freedom. Tilt or rotation may be in at least one and up to three degrees of freedom. As the hexapod drive allows a free orientation of the source in space, it can basically adjust the ultrasound and/or shockwave source to any required position and orientation.

The hexapod drive may be configured to move the ultrasound and/or shockwave source towards the patient until the exit section of the cushion touches the patient film or the patient. Such a movement helps to remove small bubbles of air within a coupling medium, e.g., an ultrasound gel or another immersion media, in the space between the top of the cushion and the patient and specifically air bubbles which may be between the top of the cushion and the patient film and/or between the patient film and the patient body. This movement is done before an ultrasound and/or shockwave treatment is started. The hexapod drive may further be configured to perform additional lateral and/or rotational movements which may help further to wipe out or remove air bubbles from the coupling gel at the top of the exit section of the cushion. If, for example, the main movement direction is along the positive z-axis, the additional movement may be in the x-y-plane defined by the x-axis and y-axis. There may also be a rotation about the z-axis.

According to the invention, the hexapod drive is configured to perform at least one lateral and/or rotational movement. Such a movement may be relative to the center axis of the ultrasound and/or shockwave source. This movement helps to wipe out or remove air bubbles from the coupling gel at the top of the exit section of the cushion. If, for example, the center axis of the ultrasound and/or shockwave source is along the positive z-axis, the movement may be in the x-y-plane defined by the x-axis and y-axis. There may also be a rotation about the z-axis.

A slight off axis component of the lateral and/or rotational movement which may result in a wobble, may improve removal of bubbles.

Further, there may be movements in alternating directions, and even reversal of direction. Also, rotations and lateral displacements may be combined at the same time or alternating.

In an embodiment, a spiral movement in an X-Y plane may be combined with a translation in Z direction towards the patient. The movement may start centered close to the center axis making circles with continuously increasing radius and moving at the same time towards the patient. The movement may start with an axial offset to the center axis. The spiral may have only one or two turns. A higher number of turns is also possible. The X-Y plane may be a plane of the patient table and the Z direction may be orthogonal thereto.

The hexapod drive may be configured to provide a spiral movement in a plane orthogonal to the center axis of the ultrasound and/or shockwave source combined with a translation in a direction of the center axis. This translation may be in a direction to a patient film and/or a patient.

A patient film may be part of the patient table.

In an embodiment, a movement along the center axis of the ultrasound and/or shockwave source, in z-direction may be done with the ultrasound and/or shock-wave source tilted slightly around an axis in the x-y-plane such that the top of the exit section of the cushion approaches the patient film or patient under an angle. When a first edge of the cushion touches or approaches the patient film or patient closer than a minimum distance, the ultrasound and/or shockwave source may be tilted, such, that it is parallel to the patient film or patient.

The hexapod drive bearing the ultrasound and/or shockwave source is also known as a hexapod platform. Such a hexapod platform also is called a Stewart platform. Basically, it is a type of parallel manipulator or parallel robot that has six linear actuators, which may be hydraulic or pneumatic jacks or electric linear actuators. Examples of electric linear actors are motors coupled to a belt or a spindle to perform a linear movement. These linear actuators are connected in pairs to three mounting positions at a base, crossing over to three mounting positions at the ultrasound and/or shockwave source. Each connection of a linear actuator to either the base or the ultrasound and/or shockwave source may include a universal joint, also called a cardan joint or a ball joint. By variation of the length of the linear actuators, the ultrasound and/or shockwave source can be moved in six degrees of freedom with respect to the base. There are three degrees of translation and three degrees of rotation. Although the use of six linear actuators is preferred, a lower number of actuators may be used, e.g., like in a delta robot with three actuators.

The hexapod drive allows to adjust the position of an ultrasound and/or shock-wave source relative to a patient's body and/or the X-ray system. Positioning of the ultrasound and/or shockwave source may be done automatically or by manual control. An automatic control may allow quick adjustment and it may also allow to store and retrieve preconfigured settings.

Such a hexapod drive is a very robust and mechanical stiff support or suspension of the ultrasound and/or shockwave source. Therefore, it can withstand high forces from the patient body, the weight of the ultrasound and/or shockwave source and dynamic loads which occur when shockwave pulses are generated. Further, a hexapod drive can be moved quickly. Therefore, a stable positioning of the ultrasound and/or shockwave source is achieved providing the additional ability to quickly correct deviations and movements by the patient.

A hexapod drive may allow movement in all 6 degrees of freedom. This allows for a precise adjustment of the position of the focal volume of the ultrasound and/or shockwave source and/or the path of the ultrasound and/or shockwave though the body of the patient.

The ultrasound and/or shockwave source is oriented in a cartesian coordinate system as follows: a y-axis may be a longitudinal axis through the center of a patient table. An x-axis may be orthogonal to the y-axis and in the plane of the table surface. A z-axis is orthogonal to the plane of the table surface and therefore orthogonal to the x-axis and the y-axis and in a direction upward from the table. There may also be a first rotation around the x-axis, a second rotation and a third rotation around the z-axis. A positive rotation may be a clockwise rotation in a direction of a positive axis.

The shock wave or ultrasound therapy system may include a system controller which may control at least the hexapod drive of the ultrasound and/or shock-wave source.

The ultrasound and/or shockwave source may be of any type suitable for generating shock waves. It may include a shock wave generator and/or transducer, which may include at least one of a coil, a spark gap or a Piezo transducer. The shock wave generator/transducer may be partially enclosed by a reflector. Depending on the type of transducer, the reflector may have a parabolic or half-elliptic shape. In case of a piezo transducer, the transducer may itself have a spherical shape, such that a reflector may not be needed. The ultrasound and/or shockwave source may have a focal volume which is distant from the ultrasound and/or shockwave source and normally around a center axis of the ultrasound and/or shockwave source. The focal volume may be defined as a volume, where the maximum shock wave intensity is maintained with a deviation of maximal -3 dB or -6 dB. If the focal volume is defined with a 6 dB deviation, the pressure at the limit of the zone is half of the maximum pressure inside the zone. The focal volume may have an elliptical shape with a length in an axial direction (defined by the center axis) of the ultrasound and/or shockwave source axis of 10 to 15 cm and a diameter between 5 and 15 mm. The focal volume normally is spaced from the shock wave generator and/or transducer.

The patient table may have a basically planar surface defining a longitudinal axis. It is configured for accommodating a patient. The ultrasound and/or shockwave source may be mounted below the patient table. In general, a shockwave source may be mounted in alternative ways, e.g., on a stand or support.

The base may be standing on a floor directly or by a stand or it may be attached to a floor. The base may also hold the table.

A method of operating an ultrasound and/or shockwave source before an ultrasound and/or shockwave treatment is started, wherein the ultrasound and/or shockwave source is suspended on a hexapod drive, includes moving the ultrasound and/or shockwave source by the hexapod drive towards a patient film or patient while at the same time performing at least one lateral and/or rotational movement.

The method may be performed with a lateral and/or rotational movement with respect to the center axis of the ultrasound and/or shockwave source.

In an embodiment, there may be an off-axis movement resulting in a wobble.

### Description of Drawings

In the following the invention will be described by way of example, without limitation of the general inventive concept, on examples of embodiment with reference to the drawings.
Figure 1 shows an embodiment of a lithotripsy system.
Figure 2 shows an embodiment of a therapy system.
Figure 3 shows a more detailed view of the coupling between ultrasound and/or shockwave source and patient.
Figure 4 shows a spiral movement pattern of the ultrasound and/or shockwave source.

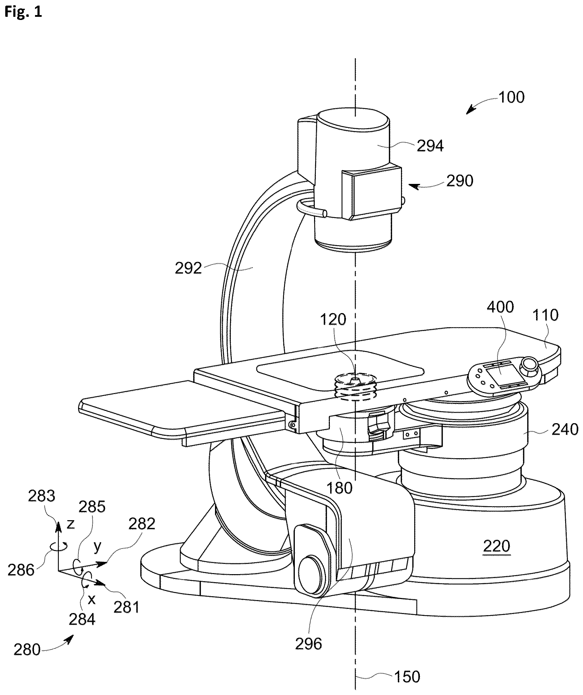

In Figure 1, a first embodiment of a lithotripsy system is shown. The invention works with any ultrasound and/or shockwave treatment system.

An extra-corporal ultrasound and/or shockwave lithotripsy system for non-invasive treatment of stones 100 comprises a patient table 110, an ultra-sound and/or shockwave source 120. The ultrasound and/or shockwave source 120 is mounted to a hexapod drive 180. The hexapod drive 180 may further be held by a stand 220. The hexapod drive 180 allows fine positioning of the ultrasound and/or shockwave source 120 in multiple axes relative to the patient table 110 and therefore relative to the patient (not shown in this figure). The ultrasound and/or shockwave source 120 has a focal volume which moves together with the source. In an embodiment the distance of the focal volume to the source may be modified.

The patient table 110 is based on a stand 220 which may stand on a floor. The table 110 may be held by a positioning device 240 to move the table relative to the ultrasound and/or shockwave source 120 together with the hexapod drive 180. A movement of the patient table may be coarse positioning which may be limited to a displacement in 3 axes.

The table 110 may have a flat surface for accommodating a patient who is not shown here.

An X-ray device 290, acting a targeting device, may be provided. It may have an X-ray tube 296 opposing an X-ray detector 294, both mounted tiltable at a C-arm 292. There is a common center axis 150 on which the X-ray device and the ultra-sound and/or shockwave source are aligned.

To describe the relative movement of the table 110, the X-ray device 290 and the ultrasound and/or shockwave source 120, a cartesian coordinate system 280 may be used. There is a y-axis 282, which may be a longitudinal axis through the center of the table. Furthermore, there is an x-axis 281 orthogonal to the y-axis and in the plane of the table surface. A z-axis 283 is orthogonal to the plane of the table surface and therefore orthogonal to the x-axis and the y-axis. There may also be a first rotation 284 around the x-axis, a second rotation 285 and a third rotation 286 around the z-axis. A positive rotation may be a clockwise rotation in a view along a positive axis.

A hexapod drive 180 may allow movement in all these 6 degrees of freedom. This allows for a precise adjustment of the position of the focal volume of the ultra-sound and/or shockwave source 120. The patient table may be positioned for a slow and coarse adjustment in 3 degrees of translation for larger distances, but normally no rotation, whereas the hexapod drive provides a comparatively quick adjustment in 3 degrees of translation and 3 degrees of rotation. The movement distances of the table may be larger than the movement distances of the hexapod drive. The X-ray device may at least be tiltable to perform a second rotation 285 around an axis parallel to the y-axis 282. It may also be tiltable around an axis parallel to the Y axis. It may further be translated in a plane defined by the X and Y axis. This means, it may be moved on the floor which is also in the X-Y plane.

For control of the movement of the ultrasound and/or shockwave source 120 relative to the patient table 110, a control panel 400 may be provided.

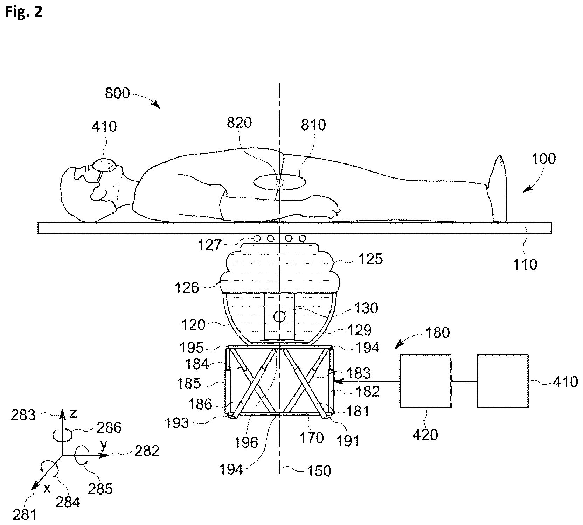

Figure 2 shows an embodiment in a schematic view of an embodiment with an ultrasound and/or shockwave source 120 mounted to a hexapod drive 180.

A shock wave or ultrasound device, which may be a lithotripter 100 may include a patient table 110 and an ultrasound and/or shockwave source 120. The ultra-sound and/or shockwave source 120 may be arranged below the patient table 110, such that a patient 800 may be accommodated on top of the patient table. The patient may have a kidney 810 with a kidney stone 820. The patient table may have a longitudinal axis. There may be a hole or cutout in the patient table at the position of the ultrasound and/or shockwave source.

Below the table 110, the ultrasound and/or shockwave source 120 is shown in a sectional view. It may have a shock wave generator 130 which may be a coil as shown herein and which is at least partially enclosed by a reflector 129. Further, the center axis 150 is marked as a dashed line. Normally, the interior of the reflector and the space between the source and the patient is filled with a liquid 126 like water or another shockwave conducting medium. To contain the water within the volume, a coupling cushion 125 may be provided. There may be small bubbles of air 127 within a coupling medium, e.g., an ultrasound gel, water, or another immersion media, in the space between the top of the cushion and the patient. Specifically, the air bubbles may be between the top of the cushion and the patient film and/or between the patient film and the patient body.

The ultrasound and/or shockwave source 120 which may have a center axis 150 is supported by and/or suspended on a hexapod drive 180. Basically, it is a type of parallel manipulator or parallel robot that has six linear actuators 181 - 186, which may be hydraulic or pneumatic jacks or electric linear actuators. These linear actuators are connected in pairs to three mounting positions 191, 192, 193 at a base 170, crossing over to three mounting positions 194, 195, 196 at the ultra-sound and/or shockwave source 120. Each connection of a linear actuator to either the base or the ultrasound and/or shockwave source may include a universal joint, also called a cardan joint or a ball joint. By variation of the length of the linear actuators, the ultrasound and/or shockwave source can be moved in six degrees of freedom with respect to the base. There are three degrees of translation, parallel to at least one X-axis 281, Y-axis 282, Z-axis 283 and three degrees of tilt or rotation including a first tilt 284 around an axis parallel to X-axis 281, a second tilt 285 around an axis parallel to Y-axis 282, a third tilt 285 around an axis parallel to Z-axis 283.

A system controller controls at least operation of the hexapod drive 180 by means of a hexapod control 420.

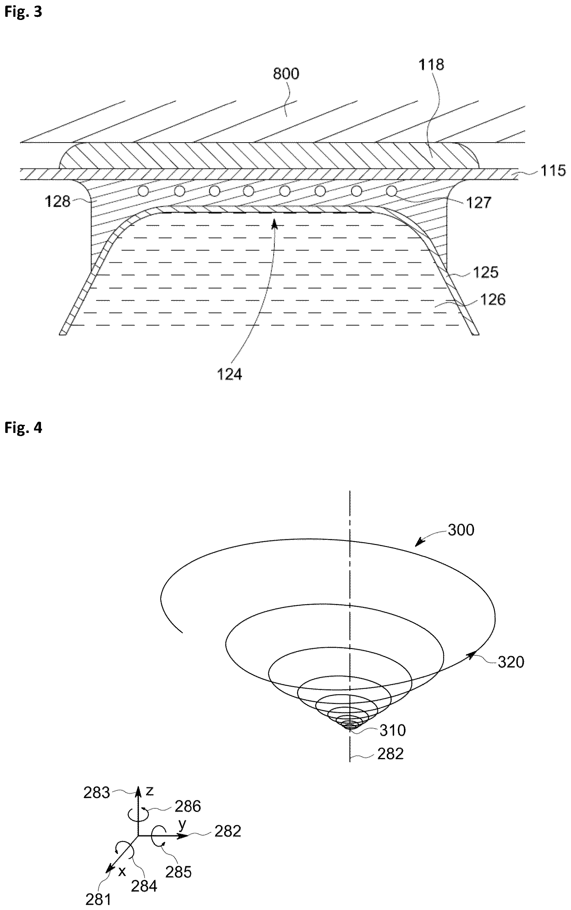

Figure 3 shows a more detailed view of the coupling between ultrasound and/or shockwave source and patient. A cushion 125 covers the interior of an ultrasound and/or shockwave source filled with a liquid 126 which may include water.

The cushion 125 may include an at least partially flexible polymer material and may have an exit section 124 through which ultrasound and/or shockwaves pass to the exterior. This exit section 124 may be directly coupled to a patient 800 or indirectly via a patient film 115 to the patient. There may be a layer of coupling gel 128, also known as ultrasound gel or acoustic gel at the top of the exit section 124 to allow coupling of the ultrasound and/or shock-wave energy. There may be another layer of coupling gel 118 or another immersion media, e.g., an ultra-sound gel, water between the patient film 115 and the patient 800. There may be a plurality of small air bubbles 127 enclosed in the coupling gel 128.

Figure 4 shows a spiral movement pattern of the ultrasound and/or shockwave source. There may be a spiral movement 300 in an X-Y plane combined with a translation in Z direction towards the patient film. The movement may start centered close to the center axis 150 making circles with continuously increasing radius and moving at the same time towards the patient film as indicated bay arrow 320. This movement may squish air bubbles in a radial direction out of the space between the cushion and the patient film. There may also be a spiral movement in a plane orthogonal to the center axis of the ultrasound and/or shockwave source combined with a translation in a direction of the center axis. This translation may be in a direction to a patient film and/or a patient. This may be slightly different to the previous movement, as the center axis of the ultrasound and/or shockwave source must not be aligned with the Z-axis.

### List of reference numerals

- 100: shock wave and/or ultrasound therapy system
- 110: patient table
- 112: longitudinal axis
- 115: patient film
- 118: coupling gel
- 120: ultrasound and/or shockwave source
- 122: coil
- 124: exit section of cushion
- 125: coupling cushion
- 126: water
- 127: air bubbles
- 128: coupling gel
- 129: reflector
- 130: shock wave generator
- 150: center axis
- 170: base
- 180: hexapod drive
- 181-186: linear actuators
- 191-193: mounting positions at base
- 194-196: mounting positions at ultrasound and/or shockwave source
- 220: stand
- 240: positioning device
- 280: cartesian coordinate system
- 281: x-axis
- 282: y-axis
- 283: z-axis
- 284: tilt around the x-axis
- 285: tilt around the y-axis
- 286: tilt around the z-axis
- 290: X-ray system
- 292: C-arm
- 293: C-arm drive
- 294: X-ray detector
- 296: X-ray tube
- 298: X-ray beam
- 300: spiral movement
- 310: spiral start point
- 320: direction of spiral movement
- 400: control panel
- 410: system controller
- 420: hexapod control
- 800: patient
- 810: kidney
- 820: kidney stone

## Claims

1. A shock wave and/or ultrasound therapy system (100) comprising an ultra-sound and/or shockwave source (120),
**characterized in that**
the ultrasound and/or shockwave source (120) is suspended on a hexapod drive (180), wherein the hexapod drive (180) is configured to provide a first movement in a direction of a center axis (150) of the ultrasound and/or shockwave source (120) and at the same time a second movement laterally and or rotationally to avoid and/or remove air bubbles at the exterior surface of a cushion covering the ultrasound and/or shockwave source.

2. The shock wave and/or ultrasound therapy system (100) according to claim 1,
**characterized in that**
the hexapod drive (180) is configured to provide a spiral movement in a plane orthogonal to the center axis (150) combined with a translation in a direction of the center axis (150).

3. The shock wave and/or ultrasound therapy system (100) according to any of the previous claims,
**characterized in that**
the hexapod drive (180) includes six linear actuators (181-186) which are attached in pairs to three positions (191-193) at the base (170), crossing over to three mounting positions (194-196) at the ultrasound and/or shockwave source (120).

4. The shock wave and/or ultrasound therapy system (100) according to any of the preceding claims,
**characterized in that**
each connection of a linear actuator (181-186) to either the base (170) or the ultrasound and/or shockwave source (120) includes a universal joint or a ball joint.

5. The shock wave and/or ultrasound therapy system (100) according to any of the preceding claims,
**characterized in that**
the linear actuators (181-186) include at least one of hydraulic or pneumatic jacks or electric linear actuators.

6. The shock wave and/or ultrasound therapy system (100) according to any of the preceding claims,
**characterized in that**
the hexapod drive (180) is configured to provide movement with at least five degrees of freedom including displacement along three orthogonal axes (281, 282, 283) and at least two degrees of tilt.

7. The shock wave and/or ultrasound therapy system (100) according to any of the preceding claims,
**characterized in that** the shock wave and/or ultrasound therapy system comprises a patient table (110);
wherein the shock wave and/or ultrasound therapy system (100) is arranged in a cartesian coordinate system with a y-axis (282) being a longitudinal axis through the center of the patient table (110), an x-axis (281) being orthogonal to the y-axis and in the plane of a surface of the patient table (110), a z-axis (283) being orthogonal to the plane of the surface of the patient table (110), and therefore orthogonal to the x-axis and the y-axis and in a direction upward from the table.

8. The shock wave and/or ultrasound therapy system (100) according to claim 7,
**characterized in that**
the ultrasound and/or shockwave source (120) is configured to generate shock waves propagating in a direction to the patient table (110) above the ultrasound and/or shockwave source (120) and having a focal volume (350) above the patient table (110).

9. A method of operating an ultrasound and/or shockwave source before an ultrasound and/or shockwave treatment is started, wherein the ultrasound and/or shockwave source is suspended on a hexapod drive, the method including moving the ultrasound and/or shock-wave source by the hexapod drive towards a patient film or a patient while at the same time performing at least one lateral and/or rotational movement.

10. The shock wave and/or ultrasound therapy system (100) according to any of the claims 7 or 8,
**characterized in that**
a patient film (115) for coupling the cushion to the patient is part of the patient table (110).

## Patentansprüche

1. Ein Stoßwellen- und/oder Ultraschalltherapiesystem (100), umfassend eine Ultraschall- und/oder Stoßwellenquelle (120),
**dadurch gekennzeichnet, dass**
die Ultraschall- und/oder Stoßwellenquelle (120) an einem Hexapod-Antrieb (180) aufgehängt ist, wobei
der Hexapod-Antrieb (180) dazu konfiguriert ist, eine erste Bewegung in Richtung einer Mittelachse (150) der Ultraschall- und/oder Stoßwellenquelle (120) und gleichzeitig eine zweite Bewegung seitlich und/oder rotatorisch bereitzustellen, um Luftblasen auf der äußeren Oberfläche eines die Ultraschall- und/oder Stoßwellenquelle bedeckenden Kissens zu vermeiden und/oder zu entfernen.

2. Das Stoßwellen- und/oder Ultraschalltherapiesystem (100) nach Anspruch 1,
**dadurch gekennzeichnet, dass**
der Hexapod-Antrieb (180) so konfiguriert ist, dass er eine Spiralbewegung in einer Ebene orthogonal zur Mittelachse (150) kombiniert mit einer Translation in Richtung der Mittelachse (150) bereitstellt.

3. Das Stoßwellen- und/oder Ultraschalltherapiesystem (100) nach einem der vorhergehenden Ansprüche,
**dadurch gekennzeichnet, dass**
der Hexapod-Antrieb (180) sechs Linearaktuatoren (181-186) umfasst, welche paarweise an drei Positionen (191-193) an der Basis (170) befestigt sind und über drei Befestigungspositionen (194-196) an der Ultraschall- und/oder Stoßwellenquelle (120) verlaufen.

4. Das Stoßwellen- und/oder Ultraschalltherapiesystem (100) nach einem der vorhergehenden Ansprüche,
**dadurch gekennzeichnet, dass**
jede Verbindung eines Linearaktuators (181-186) mit entweder der Basis (170) oder der Ultraschall- und/oder Stoßwellenquelle (120) ein Universalgelenk oder ein Kugelgelenk umfasst.

5. Das Stoßwellen- und/oder Ultraschalltherapiesystem (100) nach einem der vorhergehenden Ansprüche,
**dadurch gekennzeichnet, dass**
die Linearaktuatoren (181-186) mindestens eines von einem hydraulischen oder pneumatischen Zylinder oder elektrische Linearaktuatoren umfassen.

6. Das Stoßwellen- und/oder Ultraschalltherapiesystem (100) nach einem der vorhergehenden Ansprüche,
**dadurch gekennzeichnet, dass**
der Hexapod-Antrieb (180) so konfiguriert ist, dass er eine Bewegung mit mindestens fünf Freiheitsgraden bereitstellt, einschließlich einer Verschiebung entlang drei orthogonaler Achsen (281, 282, 283) und mindestens zwei Neigungsgraden.

7. Das Stoßwellen- und/oder Ultraschalltherapiesystem (100) nach einem der vorhergehenden Ansprüche,
**dadurch gekennzeichnet, dass**
das Stoßwellen- und/oder Ultraschalltherapiesystem einen Patiententisch (110) umfasst;
wobei das Stoßwellen- und/oder Ultraschalltherapiesystem (100) in einem kartesischen Koordinatensystem angeordnet ist, wobei eine y-Achse (282), eine Längsachse durch die Mitte eines Patiententisches (110) ist, eine x-Achse (281) orthogonal zur y-Achse und in der Ebene einer Oberfläche des Patiententisches (110) ist, eine z-Achse (283) orthogonal zur Ebene der Oberfläche des Patiententisches (110), und daher orthogonal zur x-Achse und zur y-Achse und in einer Richtung vom Tisch nach oben ist.

8. Das Stoßwellen- und/oder Ultraschalltherapiesystem (100) nach Anspruch 7,
**dadurch gekennzeichnet, dass**
die Ultraschall- und/oder Stoßwellenquelle (120) dazu konfiguriert ist, Stoßwellen zu erzeugen, die sich in Richtung des Patiententisches (110) über der Ultraschall- und/oder Stoßwellenquelle (120) ausbreiten und über dem Patiententisch (110) ein Fokusvolumen (350) aufweisen.

9. Ein Verfahren zum Betreiben einer Ultraschall- und/oder Stoßwellenquelle, bevor eine Ultraschall- und/oder Stoßwellenbehandlung beginnt, wobei die Ultraschall- und/oder Stoßwellenquelle an einem Hexapod-Antrieb aufgehängt ist, das Verfahrend einschließend
Bewegen der Ultraschall- und/oder Stoßwellenquelle durch den Hexapod-Antrieb in Richtung einer Patientenfolie oder eines Patienten bei gleichzeitigem Ausführen mindestens einer lateralen und/oder rotatorischen Bewegung.

10. Das Stoßwellen- und/oder Ultraschalltherapiesystem (100) nach einem der Ansprüche 7 oder 8,
**dadurch gekennzeichnet, dass**
eine Patientenfolie (115) zum Verbinden des Kissens mit dem Patienten Teil des Patiententisches (110) ist.

## Revendications

1. Système de thérapie par ondes de choc et/ou par ultrasons (100) comprenant une source d'ultrasons et/ou d'ondes de choc (120),
**caractérisé en ce que**
la source d'ultrasons et/ou d'ondes de choc (120) est suspendue à un organe d'entraînement hexapode (180), dans lequel
l'organe d'entraînement hexapode (180) est configuré pour fournir un premier mouvement dans une direction d'un axe central (150) de la source d'ultrasons et/ou d'ondes de choc (120) et en même temps un second mouvement latéralement et/ou de rotation pour éviter et/ou éliminer les bulles d'air à la surface extérieure d'un coussin recouvrant la source d'ultrasons et/ou d'ondes de choc.

2. Système de thérapie par ondes de choc et/ou ultrasons (100) selon la revendication 1,
**caractérisé en ce que**
l'organe d'entraînement hexapode (180) est configuré pour fournir un mouvement en spirale dans un plan orthogonal à l'axe central (150) combiné à une translation dans une direction de l'axe central (150).

3. Système de thérapie par ondes de choc et/ou ultrasons (100) selon l'une quelconque des revendications précédentes,
**caractérisé en ce que**
l'organe d'entraînement hexapode (180) inclut six actionneurs linéaires (181-186) qui sont fixés par paires à trois positions (191-193) au niveau de la base (170), passant à trois positions de montage (194-196) au niveau de la source d'ultrasons et/ou d'ondes de choc (120).

4. Système de thérapie par ondes de choc et/ou ultrasons (100) selon l'une quelconque des revendications précédentes,
**caractérisé en ce que**
chaque connexion d'un actionneur linéaire (181-186) à la base (170) ou à la source d'ultrasons et/ou d'ondes de choc (120) inclut un joint universel ou un joint à rotule.

5. Système de thérapie par ondes de choc et/ou par ultrasons (100) selon l'une quelconque des revendications précédentes,
**caractérisé en ce que**
les actionneurs linéaires (181-186) incluent au moins l'un parmi des vérins hydrauliques ou pneumatiques ou des actionneurs linéaires électriques.

6. Système de thérapie par ondes de choc et/ou par ultrasons (100) selon l'une quelconque des revendications précédentes,
**caractérisé en ce que**
l'organe d'entraînement hexapode (180) est configuré pour fournir un mouvement avec au moins cinq degrés de liberté, dont un déplacement le long de trois axes orthogonaux (281, 282, 283) et au moins deux degrés d'inclinaison.

7. Système de thérapie par ondes de choc et/ou par ultrasons (100) selon l'une quelconque des revendications précédentes,
**caractérisé en ce que** le système de thérapie par ondes de choc et/ou par ultrasons comprend une table de patient (110) ;
dans lequel
le système de thérapie par ondes de choc et/ou par ultrasons (100) est agencé dans un système de coordonnées cartésiennes dont un axe y (282) est un axe longitudinal passant par le centre de la table de patient (110), un axe x (281) est orthogonal à l'axe y et dans le plan d'une surface de la table de patient (110), un axe z (283) est orthogonal au plan de la surface de la table de patient (110), et donc orthogonal à l'axe x et à l'axe y et dans une direction allant vers le haut à partir de la table.

8. Système de thérapie par ondes de choc et/ou par ultrasons (100) selon la revendication 7,
**caractérisé en ce que**
la source d'ultrasons et/ou d'ondes de choc (120) est configurée pour générer des ondes de choc se propageant dans une direction vers la table de patient (110) au-dessus de la source d'ultrasons et/ou d'ondes de choc (120) et ayant un volume focal (350) au-dessus de la table de patient (110) .

9. Procédé d'exploitation d'une source d'ultrasons et/ou d'ondes de choc avant qu'un traitement par ultrasons et/ou ondes de choc, dans lequel la source d'ultrasons et/ou d'ondes de choc est suspendue à un organe d'entraînement hexapode, le procédé incluant la mise en mouvement de la source d'ultrasons et/ou d'ondes de choc par l'organe d'entraînement hexapode vers un film de patient ou un patient tout en effectuant en même temps au moins un mouvement latéral et/ou de rotation.

10. Système de thérapie par ondes de choc et/ou par ultrasons (100) selon l'une quelconque des revendications 7 ou 8,
**caractérisé en ce que**
un film de patient (115) destiné à coupler le coussin au patient fait partie de la table de patient (110).
